# EUROPEAN PATENT SPECIFICATION

(11) **EP 0 783 286 B1**
(45) Date of publication and mention of the grant of the patent: **10.05.2000**
(21) Application number: 95926682.6
(22) Date of filing: 14.07.1995
(51) Int. Cl.: A61F 13/15

(54) **METHOD FOR APPLYING AN ELASTIC MEMBER TO A MOVING SUBSTRATE**
VERFAHREN ZUM ANBRINGEN EINES ELASTISCHEN STREIFENS AUF EINE BEWEGTE BAHN
PROCEDE D'APPLICATION D'UN ELEMENT ELASTIQUE SUR UN SUBSTRAT EN MOUVEMENT

(30) Priority: 12.08.1994 US 289815
(43) Date of publication of application: 16.07.1997
(73) Proprietor: KIMBERLY-CLARK WORLDWIDE, INC., Neenah, Wisconsin 54956 (US)
(72) Inventor: RIBBLE, Brendon, Frank, Menasha, WI 54952 (US); VAN EPEREN, David, James, Appleton, WI 54915 (US); LAMERS, Lyle, Theodore, Appleton, WI 54915 (US)
(74) Representative: Diehl, Hermann, Dr. Dipl.-Phys.
(86) International application number: US9508822
(87) International publication number: WO9604874

(56) References cited:
- EP-A- 0 183 662
- EP-A- 0 475 419
- WO-A-89/09550
- GB-A- 2 107 573
- GB-A- 2 123 272
- US-A- 5 275 676

## Description

### Background of the Invention

### Technical Field

The present invention relates to a method for applying an elastic member to a moving substrate web. More particularly, the present invention relates to a method for applying a plurality of elastic members in a curved configuration at each of the leg opening regions of a disposable absorbent article, such as a disposable diaper (see EPA-0475419).

### Description of the Related Art

Absorbent articles, such as disposable diapers, training pants, adult incontinence articles and the like, have incorporated elasticized gathers at the leg openings of the article to help contain body exudates. The leg openings have been positioned at the lateral side margins of the article and have been elasticized with a single elastic member or with multiple elastic members. Moreover, it has been desirable to employ elastic members which are curved to better follow the contours of the leg openings formed in the side margins of disposable absorbent articles. The curved elastic members have improved the ability of the article to contain body exudates. Various techniques for applying the elastic members onto a substrate are well known to those skilled in the art. For example, a technique for applying an elastic member to a substrate web along a curved path is described in U.S. Patent No. 5,275,676 issued January 4, 1994, to Rooyakkers et al.

Many of the conventional techniques of applying elastic members on a substrate web in either a linear or curved configuration have applied an adhesive directly to the elastic members to secure the elastic members to the substrate web. For example, the adhesive has been slot-coated or sprayed on the elastic members by methods well known to those skilled in the art. Typically, the adhesive has been applied to the elastic member before it contacts the substrate web to avoid exposing the substrate to excessive amounts of heat from the adhesive which has resulted in burn-through of the substrate web. However, applying the adhesive directly to the elastic has been difficult when the elastic members are small strands and has resulted in excessive amounts of adhesive overspray which has increased waste and maintenance.

Other conventional techniques for applying elastic members on a substrate web have utilized an adhesive which has been applied to the substrate web in the form of continuous monofilaments. The continuous monofilaments of adhesive have been applied to the substrate web in a linear or swirled pattern by using directed air jets. The elastic members have then been secured to the substrate web along the continuous monofilaments. Typically, the continuous monofilaments have retained a high percentage of the heat required for formation and, thus, have also resulted in burn-through of the substrate web. In an attempt to avoid the burn-through, several conventional techniques have applied the elastic members to a carrier sheet and then bonded the carrier sheet to the substrate web. However, the addition of the carrier sheet has increased the cost and reduced the flexibility of the substrate web. Further, when a carrier sheet has been used, the elastic members have needed to be stronger to achieve the same tension characteristics and gathers in the substrate web.

Moreover, the nonuniform distribution of adhesive which has existed when using continuous monofilaments of adhesive has resulted in gaps where the elastic members have not been attached to the substrate web. As a result, the substrate web has gathered nonuniformly when the elastic members have been relaxed and the substrate web has had nonuniform tension characteristics. In addition, increased amounts of adhesive have been required to achieve the desired level of adhesion when using continuous monofilaments of adhesive. Further, in intermittent applications, the continuous monofilaments of adhesive have been broken and snap back causing the formation of a hot droplet of adhesive. Such droplets have also resulted in burn-through and have adversely affected the strength and aesthetics of the substrate web.

Thus, some of the conventional techniques of applying elastic members to a substrate web have been limited to using an adhesive applied directly to the elastic members to avoid burn-through of the substrate web. Other conventional techniques have utilized nonuniformly distributed monofilaments of adhesive applied to the substrate web which has resulted in burn-through and nonuniform tension characteristics in the resultant substrate web. The above-mentioned difficulties have been more acute when applying a plurality of elastic members, such as a plurality of elastic strands, to a substrate web in a curved configuration. Such elastic members may have a very small cross-sectional area making it difficult to apply the required amount of adhesive directly to them. Moreover, an increased adhesive force has been required to maintain the elastic members in a curved configuration versus a linear configuration.

### Summary of the Invention

In response to the discussed difficulties and problems encountered in the prior art, a new method for applying an elastic member to a substrate web and a composite material produced by the method have been discovered.

In one aspect, the present invention provides a distinctive method for intermittently applying at least one elastic member onto a continuously moving substrate web along a selected path. The substrate web is moved along a substrate path. The elastic member is supplied along an elastic path and is also elongated. A meltblown adhesive is intermittently applied on the substrate web at spaced-apart locations along the selected path. The elongated elastic member is delivered to the substrate web along the selected path and secured to the substrate web at the spaced-apart locations. Thus, a secured portion of the elongated elastic member is provided at each of the spaced-apart locations and an unsecured portion of the elongated elastic member is provided between each of the spaced-apart locations. The unsecured portions of the elongated elastic member are selectively cut and allowed to elastically contract.

In another aspect, the present invention provides a method for intermittently applying a first plurality of elastic members onto a substrate web along a first curvilinear path and a second plurality of elastic members onto the substrate web along a second curvilinear path. The substrate web is moved along a substrate path. The elastic members are supplied along an elastic path and elongated. A meltblown adhesive is intermittently applied on the substrate web at spaced-apart locations along a first selected path which includes the first curvilinear path and along a second selected path which includes the second curvilinear path. The first plurality of elongated elastic members are delivered to the substrate web along the first curvilinear path. The second plurality of elongated elastic members are delivered to the substrate web along the second curvilinear path. The elongated elastic members are secured to the substrate web at the spaced-apart locations along the first and the second curvilinear paths. Thus, a secured portion of the elongated elastic members is provided at each of the spaced-apart locations and an unsecured portion of the elongated elastic members is provided between each of the spaced-apart locations. The unsecured portions of the elongated elastic members are selectively cut and allowed to elastically contract.

In a further aspect, the present invention provides for a composite material comprising a substrate web, a meltblown adhesive and at least one elongated elastic member. The meltblown adhesive is intermittently deposited on the substrate web at spaced-apart locations along a selected path. The elongated elastic member is secured to the substrate web at the spaced-apart locations along the selected path. The composite material may be produced by any of the method aspects of the present invention. The composite material has a creep of from about 1 to about 20 millimeters at an adhesive add-on rate of about 10 grams per square meter (gsm). The composite material also requires a tension of from about 80 to about 180 grams to hold it at an elongation of 90 percent of its fully extended length at an adhesive add-on rate of about 10 grams per square meter (gsm).

In yet another aspect, the present invention provides for an absorbent article having a front portion, a back portion and a crotch portion connecting the front and back portions. The absorbent article includes a liquid-permeable bodyside liner, an outer cover, an absorbent core located between the bodyside liner and the outer cover and a pair of elasticized, longitudinally-extending leg cuffs. The leg cuffs are located at opposite longitudinal side portions of the crotch portion of the absorbent article. Each of the leg cuffs includes at least one elongated elastic member which is secured to the absorbent article along a selected path using a meltblown adhesive.

The method of the present invention can more reliably and efficiently apply an elastic member at spaced-apart locations along a selected path across the surface of a substrate web. Moreover, the method of the present invention advantageously provides a uniform distribution of adhesive which provides a composite material having uniform tension characteristics. The method of the invention can be readily adjusted to control the amount and configuration of the adhesive required to control the placement of the elastic members on the substrate web. Compared to conventional methods of applying elastic members, the present invention does not require a carrier sheet and is capable of using lower amounts of adhesive to achieve the same adhesion thereby improving both the appearance and flexibility of the substrate web and the resultant composite material. An article using the composite material produced by any of the method aspects of the invention may be perceived as having increased performance due to the uniform tension characteristics in the elastic members. The uniform tension characteristics also provide uniform gathers in the substrate web when the elastic members are allowed to retract.

### Brief Description of the Drawings

The present invention will be more fully understood and further advantages will become apparent when reference is made to the following detailed description of the invention and the drawings, in which:
Fig. 1 representatively shows a plan view of an example of a composite material of the present invention;
Fig. 2 representatively shows a plan view of another example of a composite material of the present invention;
Fig. 3 representatively shows a perspective view of one example of a method of the present invention; and
Fig. 4 representatively shows a partially cut away, plan view of a diaper article which incorporates a pair of curved elastic members for elasticizing the leg openings at the side margins of the article.

### Detailed Description of the Invention

The present invention provides a method for applying an elastic member onto a continuously moving substrate web along a selected path and a composite material produced by the same. The method is particularly useful for applying a plurality of elastic members in a curved configuration at each of the leg opening regions of a disposable absorbent article, such as a disposable diaper. It is readily apparent, however, that the method would be suitable for applying at least one elastic member along a selected path onto any substrate web or absorbent article such as adult incontinence products, feminine care products, training pants and the like.

For the purposes of the present description, the various aspects of the method of the present invention will be described as being used to apply a plurality of elastic members onto a continuously moving substrate web along two selected curvilinear paths. For example, when constructing an absorbent article, such as a disposable diaper, it may be desirable to apply a plurality of elastic members onto each of the side edge portions of the article. However, it should be readily understood that the method of the present invention can also be used to apply a single elastic member along any selected path or a plurality of elastic members along a plurality of selected paths on any substrate web. For example, the present invention may be used to apply from 1 to about 12 elastic members and desirably from about 2 to about 6 elastic members onto a substrate web along each of two curvilinear paths.

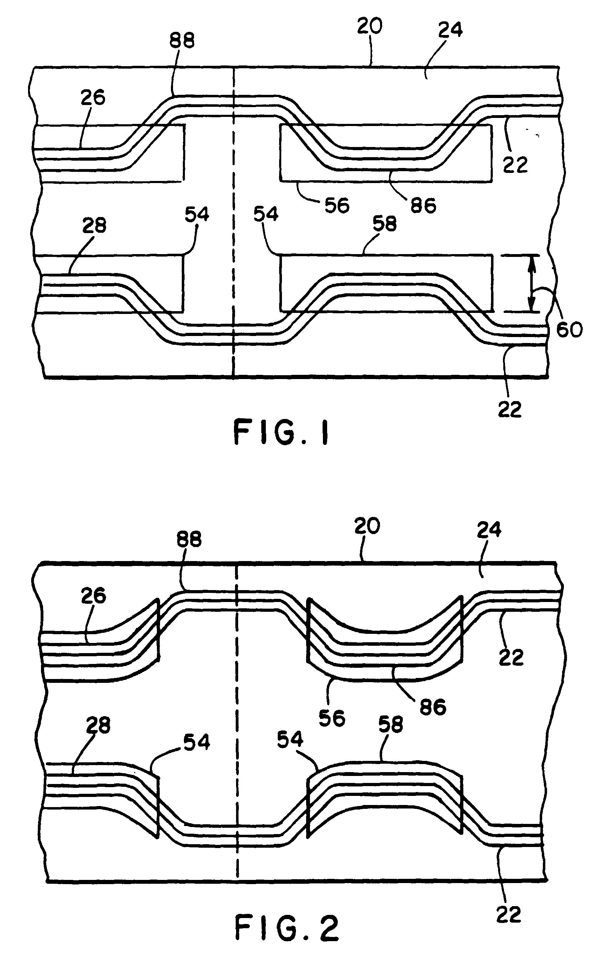

The present invention can best be understood by reference to the drawings in which like numerals represent like elements. Figs. 1 and 2 representatively illustrate a composite material 20 which includes at least one elastic member 22 secured to a substrate web 24 on both side edge regions of the substrate web 24. Desirably, the composite material 20 includes a plurality of elastic members 22 which are secured to the substrate web 24 along curvilinear paths 26 and 28. The curvilinear paths 26 and 28 may be parallel or may be independent of one another. Desirably, the curvilinear paths 26 and 28 intermittently converge and diverge from each other such that the elastic members 22 can be used to provide the leg elastics along the leg opening regions of an absorbent article. In this configuration, the elastic members 22 can be substantially symmetrically disposed relative to a longitudinal centerline of the substrate web 24. As such, the curvilinear path 26 is approximately a mirror image of the oppositely positioned curvilinear path 28. Alternatively, the composite material 20 may include at least one elastic member 22 secured to the substrate web 24 along any selected path such as, for example, a linear path. The elastic member 22 may also be continuously or intermittently applied to the substrate web 24.

The elastic member 22 suitably comprise any elastomeric material capable of being elongated at least about 50 percent, desirably from about 225 to about 400 percent, and capable of recovering to within at least about 250 percent, desirably about 150 percent of its original length after being elongated about 300 percent. In one specific embodiment, the elastic member 22 can be composed of 740 decitex Lycra® strands which are commercially available from E. I. DuPont de Nemours Corp., a company having offices located in Wilmington, Delaware. Alternatively, the elastic member 22 can be composed of a natural or synthetic rubber, a thermoplastic elastomer or a heat activatable elastic material. A wide range of materials are suitable for use as the substrate web 24. For example, the substrate web 24 can include a nonwoven material such as a spunbond, meltblown, spun laced or carded polymeric material, a film material such as a polyolefin or polyurethane film, a foam material or combinations thereof.

The composite material 20, as representatively illustrated in Figs. 1 and 2, which includes the elastic member 22 and substrate web 24 may be used in the manufacture of absorbent articles. Alternatively, the elastic member 22 can be applied directly onto an absorbent article such as a diaper, training pant, feminine care product, adult incontinence product and the like. In a specific embodiment, a plurality of elastic members 22 are selectively applied to a diaper article along selected curvilinear paths which correspond to the leg opening regions of the absorbent article.

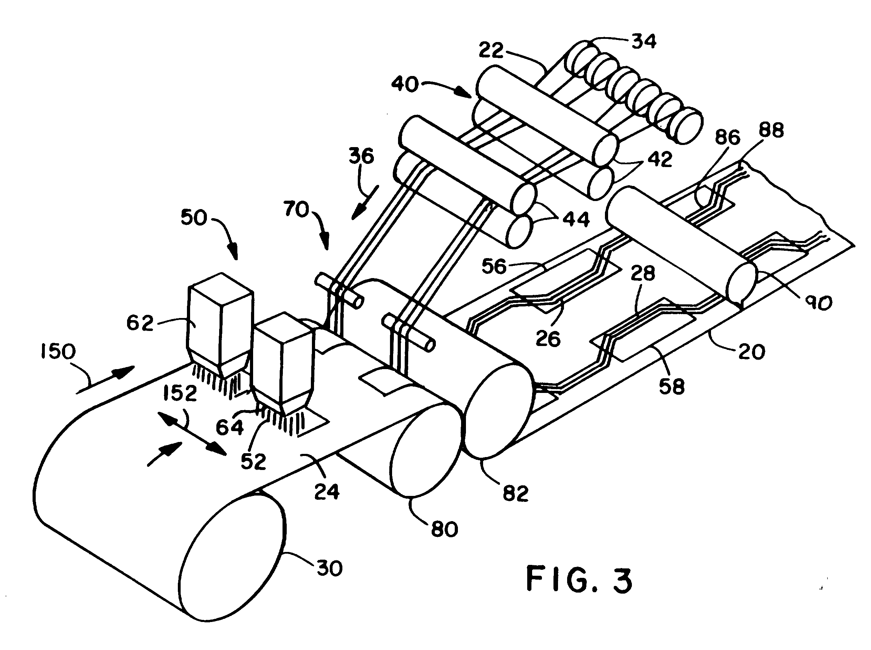

As representatively illustrated in Fig. 3, one aspect of the invention includes a transporting means 30 for moving the substrate web 24 along a substrate path 32 in a machine direction 150. At any particular location, the machine direction 150 is the direction along which the substrate web 24 is intended to move. The cross-machine direction 152 is generally perpendicular to the machine direction 150 and parallel to the plane of the substrate web 24. The z-direction is perpendicular to the plane of the substrate web 24. The transporting means 30 may be any means known to those skilled in the art such as, for example, a substrate conveyor. The present invention also may include a supplying means 34 for directing the elastic member 22 along an elastic path 36. For example, the supplying means 34 may include a pair of drive rolls 42 which may be driven by any means known to those skilled in the art, such as an electric motor. The elastic member 22 constrictively travels between the pair of drive rolls 42 and is delivered along the elastic path 36.

A tensioning means 40 for elongating the elastic member 22 may also be positioned along the elastic path 36. The tensioning means 40 may be configured to variably elongate the elastic member 22 before the elastic member is secured to the substrate web 24. As representatively illustrated in Fig. 3, the tensioning mans 40 may include two pairs of drive rolls 42 and 44 through which the elastic member 22 constrictively travels. The speed of the drive rolls 42 and 44 may be varied independently to variably elongate the elastic member 22 between the drive rolls 42 and 44. The speed of the drive rolls 42 and 44 may be controlled by any means known to those skilled in the art such as, for example, by electric motors. The tensioning means 40 can be configured to apply either a constant elongation or a variable elongation to the elastic member 22 before it is secured to the substrate web 24. For example, the elastic member 22 can be elongated from about 50 to about 400 percent, desirably from about 225 to about 325 percent and more desirably about 270 percent before it is secured to the substrate web 24. Moreover, if a plurality of elastic members 22 are used, the tensioning means 40 may variably elongate each elastic member 22 independently of each other.

As representatively illustrated in Fig. 3, an adhesive depositing means 50 may be configured to intermittently apply a meltblown adhesive 52 on the substrate web 24 at spaced-apart locations 54 in a selected deposition pattern. In a particular aspect, the meltblown adhesive 52 is intermittently applied on the substrate web 24 before the elastic member 22 contacts the substrate web 24. As used herein, reference to a "meltblown adhesive" refers to an adhesive which is applied by a meltblowing process as are well known to those skilled in the art. In general, meltblowing processes employ an extruder to force a hot melt of material such as, for example, a hot melt of adhesive, through a row of fine orifices in a meltblown die. Converging, high velocity streams of heated gas, usually air, are arranged on each side of the fine orifices. As the stream of hot melt exits the orifices, it encounters the high velocity heated gas stream, and is attenuated by the gas stream and broken into discrete fibers. The discrete fibers are then applied to the substrate web in the selected deposition pattern. The selected deposition pattern may be a linear configuration as illustrated in Fig. 1 or a curvilinear configuration as illustrated in Fig. 2. The deposition pattern may also be continuous or intermittent.

As representatively illustrated in Fig. 3, the adhesive depositing means 50 of the present invention includes at least one meltblown die 62 which has a series of fine orifices 64 through which the hot melt adhesive is forced. The meltblown die 62 can have any number of orifices 64 arranged in any configuration to achieve the desired deposition pattern of adhesive. For example, the meltblown die 62 may have from 1 to about 10 orifices per centimeter of width. The orifices 64 may be arranged in a linear configuration or in a staggered or nonlinear configuration. The orifices 64 have a diameter of from about 0.1 to about 0.5 millimeters (0.004 to about 0.020 inches) and desirably from about 0.2 to about 0.3 millimeters (0.008 to about 0.012 inches). Typically, the meltblown die 62, especially in the area of the orifices 64, is heated to provide better process control by any suitable heaters as are well known to those skilled in the art.

Any adhesive material which can be applied using a meltblowing process is suitable for use in the present invention. The selected adhesive is heated to its molten state and supplied from a conventional reservoir. Suitable adhesives include, for example, an adhesive which has the trade designation H2096 and is commercially available from Findley Adhesives Inc., a company having offices in Wauwatosa, Wisconsin, and other adhesive materials having similar properties. The adhesive is heated to a temperature sufficient to allow the molten adhesive to be pumped and extruded through the fine orifices 64 in the meltblown die 62. In one embodiment, the adhesive 52 is heated to a temperature of about 160 to about 205°C (325 - 400°F), and the molten adhesive is metered and pumped through suitable conduits to the meltblown die 62. The molten adhesive is delivered to the meltblown die 62 at a pressure of from about 7 to about 17.5 kilograms per square centimeter (100 - 250 psi). Suitable means for obtaining the pressure are well known to those skilled in the art such as, for example, a pressurizing pump. The molten adhesive is then forced through the fine orifices 64 in the meltblown die 62. In a particular aspect, the molten adhesive is forced through the orifices 64 at a flow rate of from about 10 to about 200 grams per minute, desirably from about 30 to about 100 grams per minute and more desirably from about 40 to about 90 grams per minute.

As the adhesive material 52 is forced through the fine orifices 64 in the meltblown die 62, a heated gas, such as heated air, is forced through openings located adjacent the orifices 64 in the meltblown die 62 to form selected airstreams. The heated air is typically delivered from a conventional supply of pressurized air and heated to a temperature of from about 175 to about 260°C (350 - 500°F) to provide improved process control. Typically, the heated, pressurized air flows through a pair of slots in the meltblown die 62 which extend along and are adjacent to the orifices 64 and have a slot width which is adjusted to about 0.18 centimeters (0.007 inches). The airstreams generally are supplied at a pressure of from about 0.5 to about 3.5 kilograms per square centimeter (10 - 50 psi) for improved process control. The resultant airstreams are directed towards the molten adhesive to break the adhesive stream into fine discrete fibers which are then applied to the substrate web 24 as meltblown adhesive 52.

The meltblown adhesive 52 is applied to the substrate web 24 as discrete fibers and microfibers. Typically the fibers and microfibers have a diameter of from about 10 to about 200 microns and desirably from about 10 to about 100 microns. The adhesive fibers and microfibers also have a length of from about 10 to about 50 millimeters. The diameter of the fibers will be reduced as the flow rate of the airstreams is increased.

It has been discovered that the distance between the orifices 64 in the meltblown die 62 and the substrate web 24 is an important parameter to provide the desired deposition pattern of adhesive on the substrate web 24. Accordingly, in one aspect of the invention the distance between the orifices 64 and the substrate web 24 is limited to a maximum of about 10 centimeters (about 4 inches) and is desirably from about 5 to about 7.5 centimeters (2 to about 3 inches) to provide improved control over the deposition patterns. The reduced distance between the orifices 64 and the substrate web 24 is particularly important when the desired deposition pattern of the adhesive on the substrate web 24 is a curvilinear configuration as representatively illustrated in Fig. 2.

The orifices 64 are selectively positioned in the meltblown die 62 to provide the desired deposition pattern. Desirably, as representatively illustrated in Figs. 1-3, the meltblown adhesive 52 is intermittently applied to the substrate web 24 at spaced-apart locations 54 along the substrate web 24 thereby providing adhesive areas 56 and 58 at each of the spaced-apart locations 54. The adhesive may be intermittently applied at any desired interval or spacing. Desirably, the adhesive 52 is applied at spaced-apart locations 54 that correspond to the leg opening regions of an absorbent article such that the elastic member 22 can provide the leg elastic at such locations 54.

The adhesive areas 56 and 58 may have any suitable width and length which achieves the desired adherence of the elastic member 22 to the substrate web 24. For example, as representatively illustrated in Fig. 1, the adhesive areas 56 and 58 may have a width 60 of from about 1.25 to about 12.5 centimeters (0.5 to about 5 inches) and desirably from about 2.5 to about 7.5 centimeters (1 to about 3 inches). The adhesive areas 56 and 58 may have a length of from about 5 to about 75 centimeters (2 to about 30 inches) and desirably from about 10 to about 40 centimeters (4 to about 16 inches) depending on the desired deposition pattern.

The deposition of the meltblown adhesive 52 can be intermittently controlled by any suitable means known to those skilled in the art. For example, the meltblown die 62 may include flow valves which can be independently activated to intermittently control the flow of adhesive through the orifices 64 and the resulting deposition of the meltblown adhesive 52 on the substrate. The flow valves may be controlled by any means known to those skilled in the art such as, for example, by solenoids. Electrical controls may be programed to selectively activate and deactivate the solenoids to provide the desired deposition of the meltblown adhesive 52.

The deposition of the meltblown adhesive 52 can also be selectively controlled to follow any selected path. For example, the meltblown die 62 may be oscillated in a direction essentially transverse to the substrate path 32, such as in the cross-machine direction 152, such that the meltblown adhesive 52 is deposited in a curvilinear configuration as representatively illustrated in Fig. 2. The meltblown die 62 may be oscillated by any means known to those skilled in the art such as, for example, a suitable cam mechanism. Vendors are able to design and produce suitable cam mechanisms once they are advised of particular operational parameters. Pertinent parameters can include, for example, the dimensions and inertia of the moving components, the desired number of cycles per minute, and the particular curvilinear path desired. Thus, for example, the meltblown dies 62 can be oscillated in the cross-machine direction 152 such that the meltblown adhesive 52 is intermittently applied to the substrate web 24 in a curvilinear configuration at the spaced-apart locations 54 along the curvilinear paths 26 and 28.

Alternatively, the flow of adhesive through each orifice 64 or combination of orifices across the width of the meltblown dies 62 may be selectively controlled such that the meltblown adhesive 52 is intermittently applied to the substrate web 24 along any selected path, such as the curvilinear paths 26 and 28 as representatively illustrated in Fig. 2. Thus, the flow of adhesive through the orifices 64 can be intermittently controlled to provide the desired length, width and location of the adhesive areas 56 and 58 along the selected paths. The flow of adhesive through the orifices 64 can be selectively controlled by any mans known to those skilled in the art. For example, the flow of adhesive through the orifices may be controlled using conventional flow valves controlled by solenoids which can be programmed to activate and deactivate to provide the desired deposition pattern of meltblown adhesive 52.

The meltblown adhesive 52 is generally intermittently applied to the substrate web 24 at a rate of from about 1 to about 20 grams per square meter and desirably from about 5 to about 10 grams per square meter. In a particular aspect of the invention, the meltblown adhesive 52 is applied to the substrate web 24 at a rate of no more than 10 grams per square meter to provide more improved efficiency. In a particular aspect of the invention, the meltblown adhesive 52 is intermittently applied to the substrate web 24 at each of the adhesive areas 56 and 58 at the spaced-apart locations 54 such that the adhesive is substantially uniformly distributed on the adhesive areas 56 and 58. Desirably, the meltblown adhesive 52 also substantially covers each adhesive area 56 and 58.

If it is desired to provide a composite material wherein the elastic member 22 is applied to the substrate web along a non-linear path, the different aspects of the present invention can also include an elastic oscillating means for delivering the elastic member along the desired path. As representatively illustrated in Fig. 3, an elastic oscillating means, generally indicated at 70, may be configured to guide and deliver at least one elastic member 22 to the continuously moving substrate web 24 along a selected path, such as the curvilinear paths 26 and 28. In a particular aspect, the elastic member 22 may be delivered to the curvilinear paths 26 and 28 in a curvilinear configuration which may vary through a side-to-side, traversing distance of from about 0.5 to about 7.5 centimeters measured along the substrate web 24 in the cross-machine direction 152. Any suitable means which can deliver the elastic member 22 along the desired path can be used. For example, a suitable apparatus for delivering elastic members along a curvilinear path is described in U.S. Patent 5,275,676 which issued January 4, 1994 in the name of Rooyakkers et al., and is herein incorporated by reference.

As representatively illustrated in Fig. 3, at least one rotatable nip roll, such as rotatable nip rolls 80 and 82, may be adapted to be in rolling engagement with the substrate web 24. The rotatable nip roll 80 is configured to press the elastic member 22 onto the substrate web 24 to secure the elastic member 22 on the substrate web 24 along the selected paths, such as the curvilinear paths 26 and 28. The rotatable nip roll 80 may be rotated by any means known to those skilled in the art, such as by an electric motor. In another aspect of the invention, a second substrate web may be fed into the rotatable nip roll 80 such that the elastic member 22 is disposed between two substrate webs 24.

It has been found that the securement location of the elastic member 22 onto the substrate web 24 can more closely match the desired path, such as the curvilinear paths 26 and 28, when the distance between the elastic oscillating means 70 and a position where the elastic member 22 contacts the substrate web 24 is kept to a minimum. In particular aspects of the invention, the distance between the elastic oscillating means 70 and the position where the elastic member 22 contacts the substrate web 24 is from about 0.3 to about 2.5 centimeters and, desirably, the distance is from about 0.3 to about 1.3 centimeters.

In a particular aspect of the invention wherein the meltblown adhesive is applied intermittently at spaced-apart locations 54 to the substrate web 24, the elastic member 22 is secured to the substrate web 24 at the spaced-apart locations 54 thereby providing the composite material 20. Thus, a secured portion 86 of the elastic member 22 is provided at each of the spaced-apart locations 54 and an unsecured portion 88 of the elastic member 22 is provided between each of the spaced-apart locations 54. In such a configuration, the present invention as representatively illustrated in Fig. 3, may include a blade means 90 for selectively cutting the unsecured portions 88 of the elastic member 22. The unsecured portions 88 of the elastic member may then be allowed to elastically contract.

When compared to conventional methods of applying elastic members, the amount of adhesive required in the present invention can be about 50 percent of the conventional amount of adhesive while still maintaining the desired level of adhesion and tension characteristics in the resultant composite material. Moreover, the uniform distribution of adhesive of the present invention results in the bonding of the entire length of the elastic member 22 to the substrate web 24 in the adhesive areas 56 and 58. Further, the uniform distribution of adhesive does not result in burn-through of the substrate web 24 because the uniform layer of adhesive does not retain the heat of formation as occurs when using conventional methods of applying elastic members. Thus, elastic members can be secured to a substrate web using the method of the present invention without requiring a carrier sheet.

As a result, when compared to conventional methods of applying elastic members to a substrate web, the method of the present invention can provide a composite material with maintained integrity and increased flexibility while using lower amounts of adhesive, Moreover, when compared to conventional methods of applying elastic members to a substrate web, the present invention provides a composite material with improved tension characteristics at the same adhesive add-on rates. Alternatively, the present invention can provide the same tension characteristics at lower adhesive add-on rates. For example, the composite material may require a tension of from about 80 to about 180 grams to maintain it at an elongation of 90 percent of its fully extended length at an adhesive add-on rate of 10 grams per square meter (gsm). In a particular aspect, the composite material may require a tension of 150 grams to maintain it at an elongation of 90 percent of its fully extended length at an adhesive add-on of less than 10 grams per square meter. The composite material may also have a creep of from about 1 to about 20 millimeters at an adhesive add-on of 10 grams per square meter. Thus, the composite material made by the method of the present invention has increased flexibility due to both the lack of a carrier sheet and the reduced levels of adhesive required.

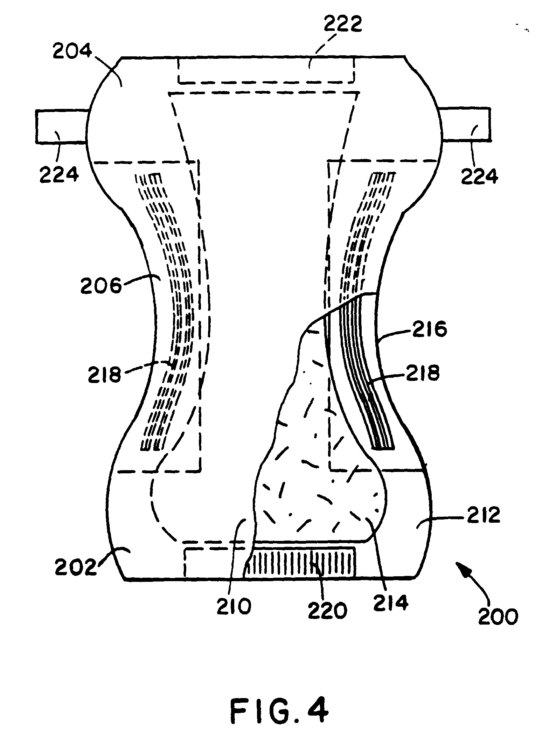

Fig. 4 representatively illustrates an absorbent article 200 which may include elastic members which can be applied using the method according to the various aspects of the present invention. The absorbent article will be described in terms of a diaper article adapted to be worn by infants about the lower torso. It is understood that the present invention is equally applicable to other absorbent articles such as adult incontinent products, training pants, feminine care products and the like. As representatively illustrated in Fig. 4, the absorbent article 200 defines a front portion 202, a rear portion 204, and a crotch portion 206 connecting the front portion 202 and the rear portion 204. The absorbent article 200 includes a bodyside liner 210, an outer cover 212 and an absorbent core 214 located between the bodyside liner 210 and the outer cover 212. As used herein, reference to a front portion refers to that part of the absorbent article which is generally located on the front of a wearer when in use. Reference to the rear portion refers to the portion of the article generally located at the rear of the wearer when in use, and reference to the crotch portion refers to that portion which is generally located between the legs of the wearer when in use.

The crotch portion 206 has opposite longitudinal side portions 208 which include a pair of elasticized, longitudinally-extending leg cuffs 216. The leg cuffs 216 are generally adapted to fit about the legs of a wearer in use and serve as a mechanical barrier to the lateral flow of body exudates. The leg cuffs 216 are elasticized by a pair of leg elastics 218. The absorbent article 200 further includes a front waist elastic 220 and a rear waist elastic 222. The rear portion 204 of the absorbent article 200 further includes a fastening means such as a pair of tape fasteners 224. The tape fasteners 224 are intended to hold the absorbent article 200 about the waist of the wearer when in use.

The bodyside liner 210 of the absorbent article 200, as representatively illustrated in Fig. 4, suitably presents a bodyfacing surface which is compliant, soft feeling and nonirritating to the wearer's skin. Further, the bodyside liner 210 may be less hydrophilic than the absorbent core 214, to present a relatively dry surface to the wearer, and may be sufficiently porous to be liquid permeable, permitting liquid to readily penetrate through its thickness. A suitable bodyside liner 210 may be manufactured from a wide selection of web materials, such as porous foams, reticulated foams, apertured plastic films, natural fibers (for example, wood or cotton fibers), synthetic fibers (for example, polyester or polypropylene fibers), or a combination of natural and synthetic fibers. The bodyside liner 210 is suitably employed to help isolate the wearer's skin from liquids held in the absorbent core 214.

Various woven and nonwoven fabrics can be used for the bodyside liner 210. For example, the bodyside liner may be composed of a meltblown or spunbonded web of polyolefin fibers. The bodyside liner may also be a bonded-carded web composed of natural and/or synthetic fibers. The bodyside liner may be composed of a substantially hydrophobic material, and the hydrophobic material may, optionally, be treated with a surfactant or otherwise processed to impart a desired level of wettability and hydrophilicity. In a particular embodiment of the present invention, the bodyside liner 210 comprises a nonwoven, spunbond, polypropylene fabric composed of about 2.8-3.2 denier fibers formed into a web having a basis weight of about 22 grams per square meter and a density of about 0.06 gram per cubic centimeter. The fabric is surface treated with about 0.28 weight percent of a surfactant commercially available from Rohm and Haas Co. under the trade designation Triton X-102.

The outer cover 212 of the absorbent article 200, as representatively illustrated in Fig. 4, may suitably be composed of a material which is either liquid permeable or liquid impermeable. It is generally preferred that the outer cover 212 be formed from a material which is substantially impermeable to liquids. For example, a typical outer cover can be manufactured from a thin plastic film or other flexible liquid-impermeable material. For example, the outer cover 212 may be formed from a polyethylene film having a thickness of from about 0.012 millimeter (0.5 mil) to about 0.051 millimeter (2.0 mils). If it is desired to present the outer cover 212 with a more clothlike feeling, the outer cover 212 may comprise a polyethylene film having a nonwoven web laminated to the outer surface thereof, such as a spunbond web of polyolefin fibers. For example, a polyethylene film having a thickness of about 0.015 millimeter (0.6 mil) may have thermally laminated thereto a spunbond web of polyolefin fibers, which fibers have a thickness of about 1.5 to 2.5 denier per filament, which nonwoven web has a basis weight of about 24 grams per square meter (0.7 ounce per square yard). Methods of forming such clothlike outer covers are known to those skilled in the art.

Further, the outer cover 212 may be formed of a woven or nonwoven fibrous web layer which has been totally or partially constructed or treated to impart a desired level of liquid impermeability to selected regions that are adjacent or proximate the absorbent core 214. Still further, the outer cover 212 may optionally be composed of a micro-porous "breathable" material which permits vapors to escape from the absorbent core 214 while still preventing liquid exudates from passing through the outer cover 212.

The absorbent core 214 of the absorbent article 200, as representatively illustrated in Fig. 4, may suitably comprise a matrix of hydrophilic fibers, such as a web of cellulosic fluff, mixed with particles of a high-absorbency material commonly known as superabsorbent material. In a particular embodiment, the absorbent core 214 comprises a mixture of superabsorbent hydrogel -forming particles and wood pulp fluff. The wood pulp fluff may be exchanged with synthetic, polymeric, meltblown fibers or with a combination of meltblown fibers and natural fibers. The superabsorbent particles may be substantially homogeneously mixed with the hydrophilic fibers or may be nonuniformly mixed. Alternatively, the absorbent core 214 may comprise a laminate of fibrous webs and superabsorbent material or other suitable means of maintaining a superabsorbent material in a localized area.

The absorbent core 214 may have any of a number of shapes. For example, the absorbent core may be rectangular, I-shaped or T-shaped. It is generally preferred that the absorbent core be narrower in the crotch portion 206 of the absorbent article 200 than in the front or rear portion, 202 or 204, respectively.

The high-absorbency material can be selected from natural, synthetic and modified natural polymers and materials. The high-absorbency materials can be inorganic materials, such as silica gels, or organic compounds, such as crosslinked polymers. The term "crosslinked" refers to any means for effectively rendering normally water-soluble materials substantially water insoluble but swellable. Such means can include, for example, physical entanglement, crystalline domains, covalent bonds, ionic complexes and associations, hydrophilic associations such as hydrogen bonding, and hydrophobic associations or Van der Waals forces.

Examples of synthetic, polymeric, high-absorbency materials include the alkali metal and ammonium salts of poly(acrylic acid) and poly(methacrylic acid), poly(acrylamides), poly(vinyl ethers), maleic anhydride copolymers with vinyl ethers and alpha-olefins, poly(vinyl pyrolidone), poly(vinyl morpholinone), poly(vinyl alcohol), and mixtures and copolymers thereof. Further polymers suitable for use in the absorbent core include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthum gum, locust bean gum, and the like. Mixtures of natural and wholly or partially synthetic absorbent polymers can also be useful in the present invention.

The high absorbency material may be in any of a wide variety of geometric forms. As a general rule, it is preferred that the high absorbency material be in the form of discrete particles. However, the high absorbency material may also be in the form of fibers, flakes, rods, spheres, needles, or the like. As a general rule, the high absorbency material is present in the absorbent core in an amount of from about 5 to about 100 weight percent based on total weight of the absorbent core 214.

The outer cover 212 and bodyside liner 210 are generally adhered to one another so as to form a pocket in which the absorbent core 214 is located. Thus, the leg cuffs 216 are suitably formed by portions of the outer cover 212, and/or bodyside liner 210, which extend beyond the longitudinal sides of the absorbent core 214. Naturally, the leg cuffs 216 can also be formed from separate materials which are attached to the outer cover 212 and/or bodyside liner 210.

The leg cuffs 216, as representatively illustrated in Fig. 4, include leg elastics 218. Materials suitable for use in forming leg elastics 218 are known to those skilled in the art. Exemplary of such materials are strands or ribbons of a polymeric, elastomeric material which are adhered to the absorbent article 200 at the leg cuffs 216 while in a stretched position, or which are attached to the absorbent article while the article is pleated, such that elastic constrictive forces are imparted to the leg cuffs 216. Waist elastics 220 and 222 and tape fasteners 224, as representatively illustrated in Fig. 4, are also known to those skilled in the art.

As representatively illustrated in Fig. 4, the leg elastics 218 may be curved to more closely fit the contours of the legs and buttocks of the wearer and better contain bodily exudates. The curved leg elastics 218 may include a plurality of elastic members that are intermittently applied to the article using the method of the various aspects of the present invention such as, for example, the method illustrated in Fig. 3. For example, the adhesive depositing means 50 of the different aspects of the present invention may intermittently apply a meltblown adhesive 52 to the outer cover 212 before the elastic members 22 are secured to the outer cover 212. After the elastic members 22 are intermittently secured to the outer cover 212, the unsecured portions of the elastic members 22 may be severed and allowed to contract. Thus, the stretched elastic members 22 may be intermittently secured to the outer cover 212 at selected locations along the curvilinear paths 26 and 28 that correspond to the leg opening regions of the absorbent article. Alternatively, instead of applying the elastic members directly to the outer cover 212 using the method of the present invention, the curved leg elastics 218 may include the composite material of the different aspects of the present invention. For example, as representatively illustrated in Fig. 2, the elastic members 22 may first be applied to the substrate web 24 in a curved configuration using a meltblown adhesive to provide a composite material 20 which may then be used to provide the leg elastics 218 of the absorbent article 200.

As discussed above, the different aspects of the present invention provide a uniform distribution of adhesive onto the substrate web or outer cover used in the absorbent article. When compared to conventional methods of applying elastic members to a substrate web, the uniform distribution of adhesive of the present invention results in more uniform gathers when the elongated elastic members are allowed to contract. The uniform gathers provide improved performance and a better fit of the absorbent article about the wearer while reducing the amount of irritation and red-marking of the wearer. The uniform distribution of adhesive also provides more complete and consistent bonding of the elastic members to the substrate web or outer cover along the entire length of the elastic members. Such consistent bonding along the entire length of the elastic members has increased importance when applying the elastic members in a curved configuration because it is more difficult to maintain the elastic members in such a configuration. Moreover, the elastic members of the present invention can be bonded directly to the substrate web or outer cover without requiring a carrier sheet. Thus, the elasticized regions of absorbent articles wherein the elastic members are applied using the different aspects of the present invention are more flexible than if conventional methods of bonding the elastic members are used. Further, in the absence of a carrier sheet, the elastic members can be smaller because they are not required to be as strong to provide the same tension characteristics in the absorbent article. Thus, the present invention provides a lower cost absorbent article by using lower levels of adhesive add-on and smaller elastic members when compared to conventional methods of applying elastic members to absorbent articles.

For the purposes of the present invention, the following test procedures are used to determine the tension characteristics of the composite material:

### Elastic Tension Test

As used herein, the term "tension" refers to the amount of force required to maintain a sample material at a specific elongation according to the following procedure. The sample, such as the composite material of the present invention, is extended by hanging a 1000 gram weight from each side edge region of the sample. A seven inch (17.8 centimeter) length of the extended sample is then designated with marks. The weights are removed and the sample is placed in a Model No. 6000 Universal tension tester which is commercially available from Chatillon, a company having offices located in Greensboro, North Carolina. The edges of the clamps of the tension tester are positioned at each mark on the sample. The portion of the sample held between the clamps is then extended to 90 percent of its fully extended length (6.3 inches or 16.0 centimeters). After a period of one minute, the force required to hold the material at 90 percent elongation is recorded. The recorded force is the tension of the sample.

### Elastic Creep Test

As used herein, the term "creep" refers to the distance that the position at which the elongated elastic member provides gathers in the substrate web contracts after a specified period of time according to the following test procedure. The sample, such as the composite material of the present invention, is extended by hanging a 1000 gram weight from each side edge region of the sample. A 6.5 inch (16.5 centimeters) length of the extended sample is then marked. The sample is then cut at the end marks. One end of the sample is taped to a flat board and the other end is extended such that the sample is extended to 75 percent of its fully extended length (4.8 inches or 12.2 centimeters) and taped in place. The positions where the elastic member begins to provide gathers in the sample are marked on both ends of the sample. The sample is placed in a 100°F oven for 90 minutes. The sample is then removed and the positions where the elastic member now provides gathers is marked on both ends of the sample. The distance the gathers have retracted is measured and recorded for both ends of the sample. The recorded distances represent the creep of the sample.

The following examples are provided for illustration purposes and are not intended to limit the scope of the present invention in any manner.

### Example 1

A plurality of elastic members are applied to both side edge regions of a continuously moving substrate web using the method of the different aspects of the present invention. The substrate web consists of a polymeric film material which has a thickness of 1 mil (0.0254 millimeters) and which is commercially available from Edison Plastics Co., a company having offices located in Macalester, Oklahoma. The substrate web is moved along at a speed of about 83.8 meters per minute (275 feet per minute). Four elastic members are supplied to each side edge region of the substrate web. Each elastic member is a 740 decitex Lycra® strand available from E. I. DuPont de Nemours Corp., a company having offices located in Wilmington, Delaware. Before being secured to the substrate web, the elastic members are elongated 270 percent. A meltblown adhesive is continuously applied to the substrate web in the desired deposition pattern along both side edge regions of the substrate web. The meltblown adhesive is a hot melt adhesive which is available under the trade designation H-2096 from Findley Adhesives, Inc., a company having offices located in Wauwatosa, Wisconsin. The meltblown adhesive is applied continuously in selected paths across a width of 3.81 centimeters (1.5 inches) along both side edge regions of the substrate web. The elongated elastic members are applied to the substrate web along the selected paths. A liner layer is provided on top of the elastic members such that the elastic members are disposed between the substrate web and the liner to provide a composite material. The elastic members are constrictively pressed between the substrate and liner layer with a rotatable nip roll to secure the elongated elastic members to the substrate web. The liner layer is a nonwoven material having a basis weight of 14.22 grams per square meter (0.6 ounces per square yard).

The meltblown adhesive is supplied by a conventional meltblowing process under the following conditions. The air used to attenuate the molten adhesive is supplied at a temperature of 238°C (460°F) and a pressure of 87.8 kilograms per square centimeter (30 psi). The temperature of the molten adhesive stored in the holding tank is maintained at 171°C (340°C). The temperature of the delivery hose and the meltblown die are maintained at 187.8°C and 193.3°C, respectively (370°F and 380°F, respectively). The diameters of the orifices in the meltblown die are about 0.25 millimeters.

The meltblown adhesive is applied to the substrate web at an add-on level of 13.7 grams per square meter. The composite material is subjected to the Elastic Creep Test and Elastic Tension Test as described above. The composite material has an average creep of 9.25 millimeters and an elastic tension of 150.9 grams.

### Example 2

A composite material is provided as set forth in Example 1 except that the meltblown adhesive is applied to the substrate web at an add-on level of 7.0 grams per square meter. The composite material is subjected to the Elastic Creep Test and Elastic Tension Test as described above. The composite material has an average creep of 18.0 millimeters and an elastic tension of 151.0 grams.

### Comparative Example

A composite material is provided as set forth in Example 1 except that continuous monofilaments of adhesive are used to secure the elongated elastic members to a pair of carrier sheets which are then secured to the substrate web. The continuous monofilaments of adhesive are applied in a swirled configuration in selected paths across a width of 1.9 centimeters (0.75 inches) along both carrier sheets. The elongated elastic members are applied to the carrier sheets along the selected paths. The carrier sheets are then secured to the side edge regions of the substrate web. A liner layer is provided on top of the elastic members such that the elastic members are disposed between the substrate web and the liner layer to provide a composite material.

The swirled monofilaments of adhesive are supplied by a conventional adhesive process under the following conditions. The air used to swirl the molten adhesive monofilaments is supplied at a temperature of 193°C (380°F) and a pressure of 1.05 kilograms per square centimeter (15 psi). The temperature of the molten adhesive stored in the holding tank is maintained at 149°C (300°F). The temperature of the delivery hose and the die are maintained at 149°C and 188°C, respectively (300°F and 370°F, respectively). The diameters of the nozzle orifices in the die are about 0.51 millimeters.

The swirled adhesive is applied to the substrate web at an add-on level of 17.0 grams per square meter. The composite material is subjected to the Elastic Creep Test and Elastic Tension Test as described above. The composite material has an average creep of 50 millimeters and an elastic tension of 135 grams.

The examples illustrate that when compared to applying elastic members to a substrate web using continuous monofilaments of adhesive, the present invention provides a composite material with improved tension characteristics at lower adhesive add-on rates. Thus, the composite material of the different aspects of the present invention has increased flexibility due to both the lack of a carrier sheet and the reduced levels of adhesive required.

Having thus described the invention in rather full detail, it is readily apparent that various changes and modifications can be made without departing from the the invention. All of such changes and modifications are contemplated as being within the scope of the present invention, as defined by the subjoined claims.

## Claims

1. A method for intermittently applying at least one elastic member onto a continuously moving substrate web along a selected path, said method comprising the steps of:
(a) moving said substrate web along a substrate path;
(b) supplying said elastic member along an elastic path;
(c) elongating said elastic member;
(d) intermittently applying a meltblown adhesive on said substrate web at spaced-apart locations along said selected path;
(e) delivering said elongated elastic member to said substrate web along said selected path;
(f) securing said elongated elastic member to said substrate web at said spaced-apart locations thereby providing a secured portion of said elongated elastic member at each of said spaced-apart locations and an unsecured portion of said elongated elastic member between each of said spaced-apart locations; and
(g) selectively cutting said unsecured portions of said elongated elastic member and allowing said unsecured portions to elastically contract, characterized in that said applying step includes the step of intermittently depositing said meltblown adhesive on said substrate web to provide an adhesive area at each of said spaced-apart locations wherein said meltblown adhesive is substantially uniformly distributed on said adhesive areas.

2. The method as recited in claim 1 wherein said supplying step includes the step of providing from about 1 to about 12 elastic members.

3. The method as recited in claim 1 wherein said elongating step includes the step of elongating said elastic member from about 50 to about 400 percent.

4. The method as recited in claim 1 wherein said applying step includes the step of intermittently depositing said meltblown adhesive on said substrate web before said elastic member contacts said substrate web.

5. The method as recited in claim 1 wherein said applying step includes the step of intermittently depositing said meltblown adhesive as adhesive fibers having a diameter of from about 10 to about 200 microns.

6. The method as recited in claim 1 wherein said applying step includes the step of intermittently depositing said meltblown adhesive onto said substrate web at a rate of from about 1 to about 20 grams per square meter.

7. The method as recited in claim 1 wherein said applying step includes the step of intermittently depositing said meltblown adhesive to substantially cover said adhesive areas.

8. The method as recited in claim 7 wherein said applying step includes the step of intermittently depositing said meltblown adhesive to provide a width of said adhesive areas of from about 1,25 to about 12.5 centimeters.

9. The method as recited in claim 1 wherein said applying step includes the step of intermittently depositing said meltblown adhesive in a curvilinear configuration at said spaced-apart locations.

10. The method as recited in claim 9 wherein said depositing step includes the steps of intermittently dispensing said meltblown adhesive through a meltblown die and oscillating said meltblown die to intermittently deposit said meltblown adhesive in said curvilinear configuration.

11. The method as recited in claim 9 wherein said depositing step includes the steps of intermittently dispensing said meltblown adhesive through a meltblown die having a series of orifices and selectively controlling said orifices to intermittently deposit said meltblown adhesive in said curvilinear configuration.

12. The method as recited in claim 1 wherein said delivering step includes the step of guiding said elastic member to deliver said elastic member along a curvilinear path.

13. A method according to claim 1 wherein a first plurality of elastic members is intermittently applied onto a substrate web along a first curvilinear path and wherein a second plurality of elastic members is intermittently applied onto said substrate web along a second curvilinear path, wherein in step (a) said substrate web is moved along a substrate path; in step (b) said first and second plurality of elastic members are supplied along an elastic path; wherein in step (c) said first and second plurality of elastic members are elongated, wherein in step (d) a meltblown adhesive is intermittently applied on said substrate web at spaced-apart locations along a frist selected path which includes said first curvilinear path and along a second selected path which includes said second curvilinear path; wherein in step (e) said first plurality of elongated elastic members are delivered to said substrate web along said first curvilinear path; further comprising (e') delivering said second plurality of elongated elastic members to said substrate web along said second curvilinear path; and wherein in step (f) said first and said second plurality of elongated elastic members are secured to said substrate web at said spaced-apart locations along said first and said second curvilinear path thereby providing a secured portion of said elongated elastic members at each of said spaced-apart locations and an unsecured portion of said elongated elastic members between each of said spaced-apart locations; and wherein in step (g) said unsecured portions of said elongated elastic member are selectively cut and said unsecured portions are allowed to elastically contract.

14. The method as recited in claim 13 wherein said supplying step includes the step of providing from 1 to about 12 elastic members.

15. The method as recited in claim 13 wherein said elongating step includes the step of elongating said first and second plurality of elastic members from about 50 to about 400 percent.

16. The method as recited in claim 13 wherein said applying step includes the step of uniformly depositing said meltblown adhesive on said substrate web at said spaced-apart locations at a rate of from about 1 to about 20 grams per square meter.

17. The method as recited in claim 13 wherein said applying step includes the step of intermittently depositing said meltblown adhesive as adhesive fibers having a diameter of from about 10 to about 200 microns.

18. The method as recited in claim 13 wherein said applying step includes the step of intermittently depositing said meltblown adhesive on said substrate web in a curvilinear configuration at said spaced-apart locations along said first and said second curvilinear paths.

19. A composite material produced by the method as recited in claim 1 wherein said meltblown adhesive is deposited on said substrate web at a rate of about 10 grams per square meter and said material has a creep of from about 1 to about 20 millimeters.

20. A composite material according to claim 19 wherein said meltblown adhesive is deposited on said substrate web at a rate of about 10 grams per square meter and said composite material requires a tension of from about 80 to about 180 grams to maintain said composite material at an elongation of 90 percent of a fully extended length of said composite material.

21. A composite material as recited in claim 19 wherein from 1 to about 12 elastic members are secured to said substrate at said spaced-apart locations.

22. The composite material as recited in claim 19 wherein said elastic member is elongated from about 50 to about 400 percent.

23. The composite material as recited in claim 19 wherein said meltblown adhesive is adhesive fibers having a diameter of from about 10 to about 200 microns.

24. The composite material as recited in claim 19 wherein said meltblown adhesive is substantially uniformly distributed on said substrate web at adhesive areas at each of said spaced-apart locations.

25. The composite material as recited in claim 24 wherein said meltblown adhesive substantially covers said adhesive areas.

26. The composite material as recited in claim 19 wherein said meltblown adhesive is arranged on said substrate web in a curvilinear configuration at said spaced-apart locations.

27. The composite material as recited in claim 19 wherein said elastic member is secured to said substrate web along a curvilinear path.

28. The composite material as recited in claim 19 wherein said meltblown adhesive is deposited on said substrate web at a rate of about 10 grams per square meter and said material has a creep of from about 1 to about 20 millimeters.

29. The composite material as recited in claim 19 wherein said meltblown adhesive is deposited on said substrate web at a rate of about 10 grams per square meter and said composite material requires a tension of from about 80 to about 180 grams to maintain said composite material at an elongation of 90 percent of a fully extended length of said composite

30. An absorbent article having a front portion, a back portion, and a crotch portion connecting said front and back portions, said crotch portion having opposite longitudinal side portions, said absorbent article comprising:
(a) a liquid-permeable bodyside liner;
(b) an outer cover;
(c) an absorbent core located between said bodyside liner and said outer cover;
(d) a pair of elasticized, longitudinally-extending leg cuffs located in said crotch portion at said opposite longitudinal side portions wherein each of said leg cuffs includes at least one composite material according to claim 19.

31. The absorbent article as recited in claim 30 wherein from 1 to about 12 elastic members are secured to said absorbent article.

32. The absorbent article as recited in claim 30 wherein said elastic member is elongated from about 50 to about 400 percent.

33. The absorbent article as recited in claim 30 wherein said meltblown adhesive is adhesive fibers having a diameter of from about 10 to about 200 microns.

34. The absorbent article as recited in claim 30 wherein said meltblown adhesive is arranged on said absorbent article in a curvilinear configuration.

35. The absorbent article as recited in claim 30 wherein said elastic member is secured to said absorbent article along a curvilinear path.

36. The absorbent article as recited in claim 30 wherein said meltblown adhesive is deposited on said absorbent article at a rate of about 10 grams per square meter and said article has a creep of from about 1 to about 20 millimeters.

37. The absorbent article as recited in claim 30 wherein said meltblown adhesive is deposited on said absorbent article at a rate of about 10 grams per square meter and said article requires a tension of from about 80 to about 180 grams to maintain said article at an elongation of 90 percent of a fully extended length of said article.

## Patentansprüche

1. Verfahren zum diskontinuierlichen Aufbringen zumindest eines elastischen Elements auf einer sich kontinuierlich bewegenden Substratbahn entlang eines ausgewählten Weges, wobei das Verfahren die folgenden Schritte umfasst:
(a) Bewegen der Substratbahn entlang eines Substratwegs;
(b) Zuführen des elastischen Elements entlang eines elastischen Wegs;
(c) Dehnen des elastischen Elements;
(d) diskontinuierliches Aufbringen eines Schmelzblashaftmittels auf die Substratbahn an beabstandeten Stellen entlang des ausgewählten Weges;
(e) Übergeben des gedehnten elastischen Elements an die Substratbahn entlang des ausgewählten Weges;
(f) Befestigen des gedehnten elastischen Elements an der Substratbahn an den beabstandeten Stellen, wodurch ein befestigter Abschnitt des gedehnten elastischen Elements an jeder der beabstandeteten Stellen zur Verfügung gestellt wird und ein unbefestigter Abschnitt des gedehnten elastischen Elements zwischen den jeweiligen beabstandeten Stellen; und
(g) selektives Schneiden der unbefestigten Abschnitte des gedehnten elastischen Elements und Erlauben der unbefestigten Abschnitte, sich elastisch zusammenzuziehen, dadurch gekennzeichnet, daß der Aufbringungsschritt den Schritt des diskontinuierlichen Ablagerns des Schmelzblashaftmittels auf der Substratbahn umfaßt, um an jeder der beabstandeten Stellen einen Haftbereich zur Verfügung zu stellen, wobei das Schmelzblashaftmittel im wesentlichen gleichmäßig über die Haftbereiche verteilt ist.

2. Verfahren gemäß Anspruch 1, wobei der Zuführschritt den Schritt des Zurverfügungstellens von etwa 1 bis etwa 12 elastischen Elementen umfasst.

3. Verfahren gemäß Anspruch 1, wobei der Dehnungsschritt den Schritt des Dehnens des elastischen Elements von etwa 50 bis etwa 400 Prozent umfasst.

4. Verfahren gemäß Anspruch 1, wobei der Aufbringschritt den Schritt des diskontinuierlichen Ablagerns des Schmelzblashaftmittels auf die Substratbahn umfasst, bevor das elastische Element die Substratbahn berührt.

5. Verfahren gemäß Anspruch 1, wobei der Aufbringschritt den Schritt des diskontinuierlichen Ablagerns das Schmelzblashaftmittels als Haftfasern umfasst, welche einen Durchmesser von etwa 10 bis etwa 200 Mikron aufweisen.

6. Verfahren gemäß Anspruch 1, wobei der Aufbringungsschritt den Schritt des diskontinuierlichen Ablagerns des Schmelzblashaftmittels auf der Substratbahn in einer Menge von etwa 1 bis etwa 20 Gramm pro Quadratmeter umfasst.

7. Verfahren gemäß Anspruch 1, wobei der Aufbringungsschritt den Schritt des diskontinuierlichen Ablagerns des Schmelzblasmittels umfasst, um im wesentlichen die Haftbereiche zu bedecken.

8. Verfahren gemäß Anspruch 7, wobei der Aufbringungschritt den Schritt des diskontinuierlichen Ablagerns des Schmelzblashaftmittels umfasst, um eine Breite der Haftbereiche von etwa 1,25 bis etwa 12,5 Zentimeter zur Verfügung zu stellen.

9. Verfahren gemäß Anspruch 1, wobei der Aufbringungschritt dem Schritt des diskontinuierlichen Ablagerns des Schmelzblashaftmittels in einer krummlinigen Konfiguration an den beabstandeten Stellen umfasst.

10. Verfahren gemäß Anspruch 9, wobei der Ablagerungsschritt den Schritt des diskontinuierlichen Abgebens des Schmelzblashaftmittels durch eine Schmelzblasdüse und Schwenken der Schmelzblasdüse umfasst, um diskontinuierlich das Schmelzblashaftmittel in der krummlinigen Konfiguration abzulagern.

11. Verfahren gemäß Anspruch 9, wobei der Ablagerungsschritt die Schritte des diskontinuierlichen Abgebens des Schmelzblashaftmittels durch eine Schmelzblasdüse umfasst, welche eine Reihe von Öffnungen aufweist, und die selektive Steuerung der Öffnungen, um diskontinuierlich das Schmelzblashaftmittel in der krummlinigen Konfiguration abzulagern.

12. Verfahren gemäß Anspruch 1, wobei der Schritt des Übergebens den Schritt des Führens des elastischen Elements umfasst, um das elastische Element entlang eines krummlinigen Weges zu übergeben.

13. Verfahren gemäß Anspruch 1, wobei eine erste Mehrzahl von elastischen Elementen diskontinuierlich auf eine Substratbahn entlang eines ersten krummlinigen Weges aufgebracht wird, und wobei eine zweite Mehrzahl von elastischen Elementen diskontinuierlich auf die Substratbahn entlang eines zweiten krummlinigen Weges aufgebracht wird, wobei in Schritt (a) die Substratbahn entlang eines Substratweges bewegt wird; in Schritt (b) die erste und die zweite Mehrzahl von elastischen Elementen entlang eines elastischen Weges zugeführt werden; wobei in Schritt (c) die erste und die zweite Mehrzahl von elastischen Elementen gedehnt werden, wobei in Schritt (d) ein Schmelzblashaftmittel diskontinuierlich auf die Substratbahn an beabstandeten Stellen entlang eines ersten ausgewählten Weges aufgebracht wird, welcher den ersten krummlinigen Weg einschließt und entlang eines zweiten ausgewählten Weges, welcher den zweiten krummlinigen Weg einschließt; wobei in Schritt (e) die erste Mehrzahl von gedehnten elastischen Elementen an die Substratbahn entlang des ersten krummlinigen Weges übergeben wird; weiter umfassend (e') das Übergeben der zweiten Mehrzahl von gedehnten elastischen Elementen auf die Substratbahn entlang des zweiten krummlinigen Weges; und wobei in Schritt (f) die erste und die zweite Mehrzahl von gedehnten elastischen Elementen an der Substratbahn an den beabstandeten Stellen entlang des ersten und des zweiten krummlinigen Weges befestigt werden, wodurch ein befestigter Abschnitt der gedehnten elastischen Elemente an jeder der beabstandeten Stellen zur Verfügung gestellt wird und ein unbefestigter Abschnitt der gedehnten elastischen Elemente zwischen den beabstandeten Stellen; und wobei in Schritt (g) die unbefestigten Abschnitte des gedehnten elastischen Elements selektiv geschnitten werden und es den unbefestigten Abschnitten ermöglicht wird, sich elastisch zusammenzuziehen.

14. Verfahren gemäß Anspruch 13, wobei der Zuführschritt den Schritt des Zurverfügungstellens von 1 bis etwa 12 elastischen Elementen umfasst.

15. Verfahren gemäß Anspruch 13, wobei der Dehnungsschritt den Schritt des Dehnens der ersten und zweiten Mehrzahl von elastischen Elementen um etwa 50 bis etwa 400 Prozent umfasst.

16. Verfahren gemäß Anspruch 13, wobei der Aufbringschritt den Schritt des gleichmäßigen Ablagerns des Schmelzblashaftmittels auf der Substratbahn an den beabstandeten Stellen bei einer Rate von etwa 1 bis etwa 20 Gramm pro Quadratmeter umfasst.

17. Verfahren gemäß Anspruch 13, wobei der Aufbringschritt den Schritt des diskontinuierlichen Ablagerns des Schmelzblashaftmittels als Haftfasern umfasst, welche einen Durchmesser von etwa 10 bis etwa 200 Micron aufweisen.

18. Verfahren gemäß Anspruch 13, wobei der Aufbringungsschritt den Schritt des diskontinuierlichen Ablagerns des Schmelzblashaftmittels auf der Substratbahn in einer krummlinigen Konfiguration an den beabstandeten Stellen entlang der ersten und der zweiten krummlinigen Wege umfasst.

19. Verbundmaterial, hergestellt nach dem Verfahren gemäß Anspruch 1, wobei das Schmelzblashaftmittel auf der Substratbahn bei einer Rate von etwa 10 Gramm pro Quadratmeter abgelagert wird, und wobei das Material ein Kriechen von etwa 1 bis etwa 20 Millimeter aufweist.

20. Verbundmaterial gemäß Anspruch 19, wobei das Schmelzblashaftmittel auf die Substratbahn bei einer Rate von etwa 10 Gramm pro Quadratmeter abgelagert wird, und wobei das Verbundmaterial eine Spannung von etwa 80 bis etwa 180 Gramm benötigt, um das Verbundmaterial bei einer Dehnung von 90% einer voll ausgedehnten Länge des Verbundmaterials zu halten.

21. Verbundmaterial gemäß Anspruch 19, wobei 1 bis 12 elastische Elemente auf dem Substrat an den beabstandeten Stellen befestigt werden.

22. Verbundmaterial gemäß Anspruch 19, wobei das elastische Element von etwa 50 bis etwa 400% gedehnt wird.

23. Verbundmaterial gemäß Anspruch 19, wobei es sich bei dem Schmelzblashaftmittel um Haftfasern handelt, welche einen Durchmesser von etwa 10 bis etwa 200 Micron aufweisen.

24. Verbundmaterial gemäß Anspruch 19, wobei das Schmelzblashaftmittel im wesentlichen gleichmäßig auf der Substratbahn an Haftbereichen an den jeweiligen beabstandeten Stellen verteilt ist.

25. Verbundmaterial gemäß Anspruch 24, wobei das Schmelzblashaftmittel im wesentlichen die Haftbereiche bedeckt.

26. Verbundmaterial gemäß Anspruch 19, wobei das Schmelzblashaftmittel auf der Substratbahn in einer krummlinigen Konfiguration an den beabstandeten Stellen angeordnet ist.

27. Verbundmaterial gemäß Anspruch 19, wobei das elastische Element an der Substratbahn entlang eines krummlinigen Weges befestigt ist.

28. Verbundmaterial gemäß Anspruch 19, wobei das Schmelzblashaftmittel auf der Substratbahn bei einer Rate von etwa 10 Gramm pro Quadratmeter abgelagert ist, und wobei das Material ein Kriechen von etwa 1 bis etwa 20 Millimeter aufweist.

29. Verbundmaterial gemäß Anspruch 19, wobei das Schmelzblashaftmittel auf der Substratbahn bei einer Rate von etwa 10 Gramm pro Quadratmeter abgelagert ist, und wobei das Verbundmaterial eine Spannung von etwa 80 bis etwa 180 Gramm benötigt, um das Verbundmaterial bei einer Dehnung von 90% einer voll ausgedehnten Länge des Verbundmaterials zu halten.

30. Saugfähiger Artikel, der einen Vorderabschnitt, einen Rückabschnitt und einen Schrittabschnitt umfasst, der den Vorderabschnitt und den Rückabschnitt verbindet, wobei der Schrittabschnitt gegenüberliegende longitudinale Seitenbereiche aufweist, wobei der saugfähige Artikel aufweist:
(a) eine flüssigkeitsdurchlässige körperseitige Lage;
(b) eine äußere Abdeckung;
(c) einen saugfähigen Kern, der zwischen der körperseitigen Lage und der äußeren Abdeckung angeordnet ist;
(d) ein Paar elastische, sich longitudinal erstreckende Beinmanschetten im Schrittabschnitt an den gegenüberliegenden longitudinalen Seitenbereichen, wobei jede der Beinmanschetten mindestens ein Verbundmaterial gemäß Anspruch 19 umfasst.

31. Saugfähiger Artikel gemäß Anspruch 30, wobei 1 bis etwa 12 elastische Elemente an dem saugfähigen Artikel befestigt sind.

32. Saugfäiger Artikel gemäß Anspruch 30, wobei das elastische Element etwa 50 bis etwa 400% gedehnt ist.

33. Saugfähiger Artikel gemäß Anspruch 30, wobei es sich bei dem Schmelzblashaftmittel um Haftfasern handelt, welche einen Durchmesser von etwa 10 bis etwa 200 Micron aufweisen.

34. Saugfähiger Artikel gemäß Anspruch 30, wobei das Schmelzblashaftmittel auf dem saugfähigen Artikel in einer krummlinigen Konfiguration angeordnet ist.

35. Saugfähiger Artikel gemäß Anspruch 30, wobei das elastische Element an dem saugfähigen Artikel entlang eines krummlinigen Weges befestigt ist.

36. Saugfähiger Artikel gemäß Anspruch 30, wobei das Schmelzblashaftmittel auf dem saugfähigen Artikel bei einer Rate von etwa 10 Gramm pro Quadratmeter abgelagert ist, und wobei der Artikel ein Kriechen von etwa 1 bis etwa 20 Millimeter aufweist.

37. Saugfähiger Artikel gemäß Anspruch 30, wobei das Schmelzblashaftmittel auf dem saugfähigen Artikel bei einer Rate von etwa 10 Gramm pro Quadratmeter abgelagert ist, und wobei der Artikel eine Spannung von etwa 80 bis etwa 180 Gramm benötigt, um den Artikel bei einer Dehnung von 90% einer voll ausgedehnten Länge des Artikels zu halten.

## Revendications

1. Procédé pour appliquer par intermittence au moins un élément élastique sur une nappe de substrat se déplaçant en continu le long d'une trajectoire sélectionnée, ledit procédé comprenant les étapes suivantes qui consistent à:
(a) déplacer ladite nappe de substrat le long d'une trajectoire de substrat ;
(b) fournir ledit élément élastique le long d'une trajectoire d'élastique ;
(c) allonger ledit élément élastique ;
(d) appliquer par intermittence un adhésif obtenu par fusion-soufflage sur ladite nappe de substrat au niveau d'emplacements espacés par des intervalles le long de ladite trajectoire sélectionnée ;
(e) délivrer ledit élément élastique allongé à ladite nappe de substrat le long de ladite trajectoire sélectionnée ;
(f) fixer ledit élément élastique allongé à ladite nappe de substrat au niveau desdits emplacements espacés par des intervalles et ainsi obtenir une portion fixée dudit élément élastique allongé au niveau de chaque emplacement séparé par des intervalles et une portion non fixée dudit élément élastique allongé entre chaque emplacement séparé par des intervalles ; et
(g) couper de manière sélective lesdites portions non fixées dudit élément élastique allongé et permettre auxdites portions non fixées de se contracter de manière élastique, caractérisé en ce que ladite étape d'application inclut une étape de dépôt par intermittence dudit adhésif obtenu par fusion-soufflage sur ladite nappe de substrat pour offrir une zone adhésive au niveau de chaque emplacement séparé par des intervalles, procédé dans lequel ledit adhésif obtenu par fusion-soufflage est sensiblement distribué de manière uniforme sur lesdites zones adhésives.

2. Procédé selon la revendication 1, dans lequel ladite étape de fourniture inclut l'étape de fourniture d'environ 1 à environ 12 éléments élastiques.

3. Procédé selon la revendication 1, dans lequel ladite étape d'élongation inclut l'étape d'élongation dudit élément élastique d'environ 50 à environ 400 %.

4. Procédé selon la revendication 1, dans lequel ladite étape d'application inclut l'étape de dépôt par intermittence dudit adhésif obtenu par fusion-soufflage sur ladite nappe de substrat avant que ledit élément élastique n'entre en contact avec ladite nappe de substrat.

5. Procédé selon la revendication 1, dans lequel ladite étape d'application inclut l'étape de dépôt par intermittence dudit adhésif obtenu par fusion-soufflage sous la forme de fibres adhésives ayant un diamètre d'environ 10 à environ 200 microns.

6. Procédé selon la revendication 1, dans lequel ladite étape d'application inclut l'étape de dépôt par intermittence dudit adhésif obtenu par fusion-soufflage sur ladite nappe de substrat à un taux d'environ 1 à environ 20 g/m².

7. Procédé selon la revendication 1, dans lequel ladite étape d'application inclut l'étape de dépôt par intermittence dudit adhésif obtenu par fusion-soufflage pour couvrir sensiblement ladite zone adhésive.

8. Procédé selon la revendication 7, dans lequel ladite étape d'application inclut l'étape de dépôt par intermittence dudit adhésif obtenu par fusion-soufflage pour obtenir une largeur desdites zones adhésives d'environ 1,25 à environ 12,5 cm.

9. Procédé selon la revendication 1, dans lequel ladite étape d'application inclut l'étape de dépôt par intermittence dudit adhésif obtenu par fusion-soufflage selon une configuration curviligne au niveau desdits emplacements espacés par des intervalles.

10. Procédé selon la revendication 9, dans lequel ladite étape de dépôt inclut les étapes de distribution par intermittence dudit adhésif obtenu par fusion-soufflage à travers une filière de fusion-soufflage et d'oscillation de ladite filière de fusion-soufflage pour déposer par intermittence ledit adhésif obtenu par fusion-soufflage selon ladite configuration curviligne.

11. Procédé selon la revendication 9, dans lequel ladite étape de dépôt inclut les étapes de distribution par intermittence dudit adhésif obtenu par fusion-soufflage à travers une filière de fusion-soufflage ayant une série d'orifices et de commande sélective desdits orifices pour déposer par intermittence ledit adhésif obtenu par fusion-soufflage selon ladite configuration curviligne.

12. Procédé selon la revendication 1, dans lequel ladite étape de distribution inclut l'étape de guidage dudit élément élastique pour délivrer ledit élément élastique le long d'une trajectoire curviligne.

13. Procédé selon la revendication 1, dans lequel un premier groupe d'éléments élastiques est appliqué par intermittence sur une nappe de substrat le long d'une première trajectoire curviligne et dans lequel un second groupe d'éléments élastiques est appliqué par intermittence sur ladite nappe de substrat le long d'une seconde trajectoire curviligne, procédé dans lequel, à l'étape (a), ladite nappe de substrat est déplacée le long d'une trajectoire de substrat ; à l'étape (b), lesdits premier et second groupes d'éléments élastiques sont fournis le long de la trajectoire d'élastique ; a l'étape (c), lesdits premier et second groupes d'éléments élastiques sont allongés ; à l'étape (d), un adhésif obtenu par fusion-soufflage est appliqué par intermittence sur ladite nappe de substrat au niveau d'emplacements séparés par des intervalles le long d'une première trajectoire sélectionnée qui inclut ladite première trajectoire curviligne et le long d'une seconde trajectoire sélectionnée qui inclut ladite seconde trajectoire curviligne ; à l'étape (e), ledit premier groupe d'éléments élastiques allongés est fourni à ladite nappe de substrat le long de la première trajectoire curviligne ; ledit procédé comprenant de plus une étape (e') de fourniture dudit second groupe d'éléments élastiques allongés à ladite nappe de substrat le long de ladite seconde trajectoire curviligne ; procédé dans lequel, à l'étape (f), ledit premier et ledit second groupes d'éléments élastiques allongés sont fixés à ladite nappe de substrat au niveau desdits emplacements espacés par des intervalles le long de ladite première et de ladite seconde trajectoires curvilignes fournissant ainsi une portion fixée dudit élément élastique allongé au niveau de chacun desdits emplacements espacés par des intervalles et une portion non fixée desdits éléments élastiques allongés entre chacun desdits emplacements espacés par des intervalles ; et, à l'étape (g), lesdites portions non fixées desdits éléments élastiques allongés sont coupées de manière sélective et lesdites portions non fixées sont autorisées à se contracter de manière élastique.

14. Procédé selon la revendication 13, dans lequel ladite étape de fourniture inclut l'étape de fourniture de 1 à environ 12 éléments élastiques.

15. Procédé selon la revendication 13, dans lequel ladite étape d'élongation inclut l'étape d'élongation desdits premier et second groupes d'éléments élastiques d'environ 50 à environ 400 %

16. Procédé selon la revendication 13, dans lequel ladite étape d'application inclut l'étape de dépôt uniforme dudit adhésif obtenu par fusion-soufflage sur ladite nappe de substrat au niveau desdits emplacements séparés par des intervalles à un taux d'environ 1 à environ 20 g/m².

17. Procédé selon la revendication 13, dans lequel ladite étape d'application inclut l'étape de dépôt par intermittence dudit adhésif obtenu par fusion-soufflage sous la forme de fibres adhésives ayant un diamètre d'environ 10 à environ 200 microns.

18. Procédé selon la revendication 13, dans lequel ladite étape d'application inclut l'étape de dépôt par intermittence dudit adhésif obtenu par fusion-soufflage sur ladite nappe de substrat selon une configuration curviligne au niveau desdits emplacements espacés par des intervalles le long de ladite première et de ladite seconde trajectoires curvilignes.

19. Matériau composite produit par le procédé selon la revendication 1, dans lequel ledit adhésif obtenu par fusion-soufflage est déposé sur ladite nappe de substrat à un taux d'environ 10 g/m² et ledit matériau a un fluage d'environ 1 à environ 20 mm.

20. Matériau composite selon la revendication 19, dans lequel ledit adhésif obtenu par fusion-soufflage est déposé sur ladite nappe de substrat à un taux d'environ 10 g/m² et ledit matériau composite requiert une tension d'environ 80 à environ 180 g pour maintenir ledit matériau composite à une élongation de 90 % dudit matériau composite totalement étendu.

21. Matériau composite selon la revendication 19, dans lequel de 1 à environ 12 éléments élastiques sont fixés audit substrat au niveau desdits emplacements séparés par des intervalles.

22. Matériau composite selon la revendication 19, dans lequel ledit élément élastique est allongé d'environ 50 à environ 400 %.

23. Matériau composite selon la revendication 19, dans lequel ledit adhésif obtenu par fusion-soufflage consiste en des fibres adhésives ayant un diamètre d'environ 10 à environ 200 microns.

24. Matériau composite selon la revendication 19, dans lequel ledit adhésif obtenu par fusion-soufflage est distribué de manière sensiblement uniforme sur ladite nappe de substrat en des zones adhésives au niveau de chacun desdits emplacements séparés par des intervalles.

25. Matériau composite selon la revendication 24, dans lequel ledit adhésif obtenu par fusion-soufflage recouvre sensiblement lesdites zones adhésives.

26. Matériau composite selon la revendication 19, dans lequel ledit adhésif obtenu par fusion-soufflage est disposé sur ladite nappe de substrat selon une configuration curviligne au niveau desdits emplacements espacés par des intervalles.

27. Matériau composite selon la revendication 19, dans lequel ledit élément élastique est fixé à ladite nappe de substrat le long d'une trajectoire curviligne.

28. Matériau composite selon la revendication 19, dans lequel ledit adhésif obtenu par fusion-soufflage est déposé sur ladite nappe de substrat à un taux d'environ 10 g/m² et ledit matériau a un fluage d'environ 1 à environ 20 mm.

29. Matériau composite selon la revendication 19, dans lequel ledit adhésif obtenu par fusion-soufflage est déposé sur ladite nappe de substrat à un taux d'environ 10 g/m² et ledit matériau composite requiert une tension d'environ 80 à environ 180 g pour maintenir ledit matériau composite à une élongation de 90 % de la longueur dudit composite totalement étendu.

30. Article absorbant ayant une portion avant, une portion arrière et une portion d'entrejambe connectant lesdites portions avant et arrière, ladite portion d'entrejambe ayant des portions latérales longitudinales opposées, ledit article absorbant comprenant :
(a) une doublure côté corporel perméable aux liquides ;
(b) une enveloppe extérieure ;
(c) un noyau absorbant situé entre ladite doublure côté corporel et ladite enveloppe extérieure ;
(d) une paire de bords de jambe élastiqués, s'étendant longitudinalement, situés dans ladite portion d'entrejambe au niveau desdites portions latérales longitudinales opposées, article dans lequel chacun desdits bords de jambe inclut au moins un matériau composite selon la revendication 19.

31. Article absorbant selon la revendication 30, dans lequel de 1 à environ 12 éléments élastiques sont fixés audit article absorbant.

32. Article absorbant selon la revendication 30, dans lequel ledit élément élastique est allongé d'environ 50 à environ 400 %.

33. Article absorbant selon la revendication 30, dans lequel ledit adhésif obtenu par fusion-soufflage consiste en des fibres adhésives ayant un diamètre d'environ 10 à environ 200 microns.

34. Article absorbant selon la revendication 30, dans lequel ledit adhésif obtenu par fusion-soufflage est disposé sur ledit article absorbant dans une configuration curviligne.

35. Article absorbant selon la revendication 30, dans lequel ledit élément élastique est fixé audit article absorbant le long d'une trajectoire curviligne.

36. Article absorbant selon la revendication 30, dans lequel ledit adhésif obtenu par fusion-soufflage est déposé sur ledit article absorbant à un taux d'environ 10 g/m² et ledit article a un fluage d'environ 1 à environ 20 mm.

37. Article absorbant selon la revendication 30, dans lequel ledit adhésif obtenu par fusion-soufflage est déposé sur ledit article absorbant à un taux de 10 g/m² et ledit article requiert une tension d'environ 80 à environ 180 g pour maintenir ledit article à une élongation de 90 % de la longueur dudit article totalement étendu.
